# EUROPEAN PATENT SPECIFICATION

(11) **EP 1 213 997 B1**
(45) Date of publication and mention of the grant of the patent: **15.06.2005**
(21) Application number: 00938696.2
(22) Date of filing: 29.05.2000
(51) Int. Cl.: A61B 17/00, A61F 13/26

(54) **ACCESSORY FOR A TRANSANAL ACCESS SURGICAL INSTRUMENT AND, IN PARTICULAR, FOR A MECHANICAL CIRCULAR SUTURING DEVICE**
ZUBEHÖR FÜR EIN TRANSANAL-INVASIV ZU VERWENDENDES, CHIRURGISCHES INSTRUMENT, INSBESONDERE FÜR EIN MECHANISCHES RUNDNAHTGERÄT
ACCESSOIRE POUR INSTRUMENT CHIRURGICAL A ACCES TRANSANAL, ET EN PARTICULIER POUR DISPOSITIF MECANIQUE A SUTURES CIRCULAIRES

(30) Priority: 20.09.1999 EP 99830590
(43) Date of publication of application: 19.06.2002
(73) Proprietor: Sapi Med S.p.A., 15100 Alessandria (IT)
(72) Inventor: ODDENINO, Gian Paolo, I-15100 Alessandria (IT)
(74) Representative: Siniscalco, Fabio
(86) International application number: PCT/EP2000/004866
(87) International publication number: WO 2001/021075

(56) References cited:
- US-A- 3 347 234
- US-A- 5 404 870

## Description

The subject of the present invention is an accessory for a transanal access surgical instrument, and, in particular, for a mechanical circular suturing device.

An accessory of this type is known from US-A-5 404 870.

Surgical instruments, such as for example a mechanical circular suturing device, which makes it possible to operate in the intestinal tract, such as for example an ampulla of rectum, passing through the anal orifice are known.

On the operative extremity of this blunt-shaped instrument there is a pointed, shaped or ogival, removable portion or head, which allows painless introduction of said extremity into the intestinal tract. In certain operations, such as for example removal of a cylindrical section of rectal mucosa, the operative extremity of said instrument is inserted separately from the head. It must however be considered that since said shape is blunt its insertion into the anal orifice is particularly traumatic.

This invention is based is the proposal of an accessory for a transanal access surgical instrument, which has structural and functional characteristics which will overcome the abovementioned problems connected with the known technique.

Said problem is resolved by means of an accessory for a transanal access surgical instrument and, in particular, for a circular, mechanical suturing device, according to Claim 1.

In order to better understand the invention, one of its non-limiting embodiment is described below and illustrated in the enclosed drawings, in which:
Figure 1 shows a top view of a first accessory to be used for the preparation of an operation to be carried out using a transanal access surgical instrument;
Figure 2 shows a front view of the accessory in Figure 1;
Figure 3 shows a perspective view of the accessory in Figure 1;
Figure 4 shows a perspective view of a second accessory to be used in combination with the first accessory;
Figure 5 shows a lateral view of the accessory in Figure 4;
Figure 6 shows a perspective view of a third accessory according to the present invention;
Figure 7 shows a lateral section of the accessory in Figure 6;
Figure 7a shows a lateral section of the detail of Figure 7, marked by the arrow VIIa;
Figure 8 shows a perspective view of the accessory in Figures 1 to 3 during an initial stage of an operation in a section of ampulla of rectum;
Figure 9 shows a partially sectioned perspective view of the section of ampulla of rectum as in Figure 8 during a second stage of the operation;
Figures 10, 11 and 12 show perspective views of successive stages of use of the accessory in Figures 6 and 7, co-operating with a mechanical circular suturing device;
Figure 13 shows a cross section of a third stage of the operation in the intestinal tract;
Figure 14 shows a cross section of a further stage of the operation in the section of ampulla of rectum; and
Figure 15 shows a sectioned perspective view of the section of ampulla of rectum after the operation.

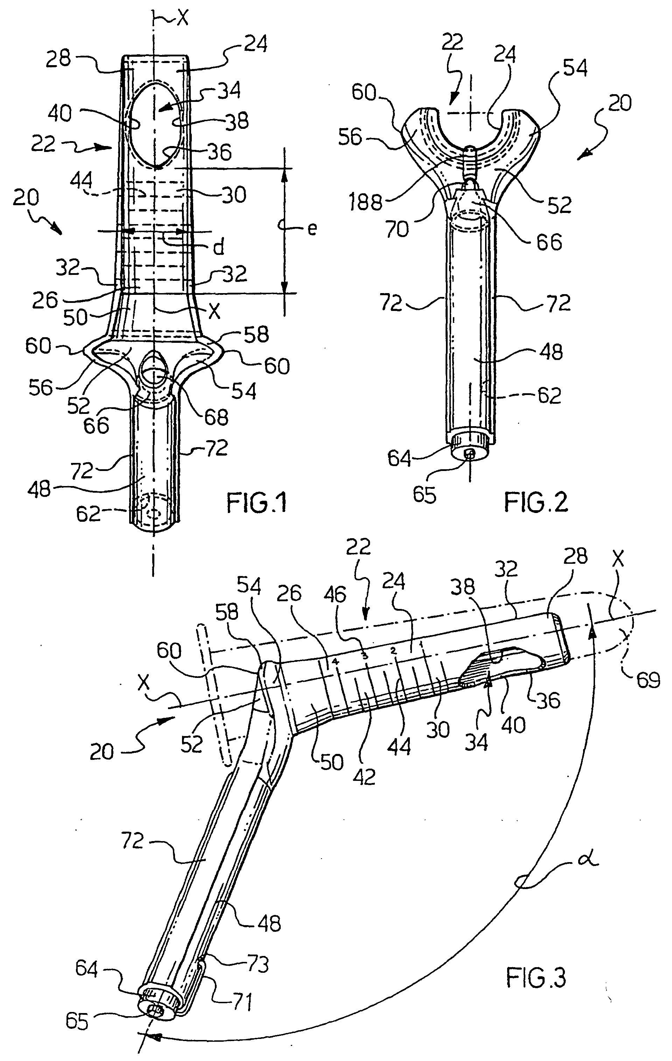

With reference to the above figures, number 20 globally indicates a first accessory to be used in the preparation stages for an operation to be effected using a transanal access surgical instrument. Said accessory includes a valve 20. Said valve 20 has an active portion 22 which is douche shaped or, in other words, rounded, or in yet other words, channelled 24 on axis X - X, which has one side convex (back) and one side concave. Said channel 24 has a semi-circular cross section. According to a preferred version of the invention said channel 24 is tapered to a first extremity 26 and a second extremity 28. The cross section diminishes in diameter or width "d" passing through a first and a second extremity 26, 28. According to a further embodiment of the invention, the wall 30 of said channel 24 diminishes in thickness passing from the first to the second of said extremities 26 and 28. The borders 32 of the channel 24 are rounded, in order to eliminate sharp edges (Figures 1 and 3).

In proximity to said second extremity 28, the wall 30 of the valve 20 is provided with an opening or window 34. Said window 34 is positioned centrally to the channel 24 and has a rounded edge 36. The edge 36 of said window 34 has a generically drawn out shape, for example elliptical, or, advantageously, it is polygonal or, with a further advantage, it is rhomboidal. The edges of the polygonal window 34 are blended or rounded. Said window 34 is extended principally in a longitudinal direction with respect to the valve 20, according to the direction of the axis X - X. Preferably, the polygonal edge 36 of the window 34 shows two opposing areas or sections 38 and 40 arranged parallely and longitudinally to the channel 24. Said window 34 is provided at a set distance "e" from the first extremity 26 of the channel 24. The external surface 42 of the back of the channel preferably shows a graduated scale 44. Said graduated scale 44 is positioned in the section of the channel which separates the window 34 from the first extremity 26. Said graduated scale 44 is flanked by numerical indications 46 showing the distance from the edge 36 of the window 34. For example, said graduated scale 44 and said numerical indications 46 are in relief on the wall 30 of the valve 20.

The first extremity 26 of the channel 24 is connected with a handle or a grip 48. The axis of said grip 48 forms an angle "α" with the active channel portion 22, 24. Thus the grip 48 is distanced from the X - X axis of the active portion 22. For example, the grip 48 forms an angle "α" with the active portion 22 between 100 degrees and 145 degrees and, preferably equal to 120 degrees. According to a particular form of realization, said extremity 26 joins a section of coaxially positioned conic channel 50. Said conic section 50 is connected with a section of tapered channel 52 which is in turn connected to the grip 48 passing through a section that splays into two wings 54 and 56 or, in other words, a stretch in which channel of the funnel-shaped section 52 reaches its maximum expansion to then close suddenly on the grip 48. The edge 58 of the channel of the funnel-shaped section 52, in the point at which the widened section shows the sudden variation in width, has a point 60 or guard for the valve 20.

The grip 48 can be associated with a means of illumination 64. According to a preferred version of the invention, the grip 48 is cylindrical and housed by a seat 62. Said seat 62 accepts said removable means of illumination 64. Preferably the grip 48 is tubular and the cavity of said tubular grip forms the seat 62 for the means of illumination 64. Said seat 62 has a tapered or truncated conical terminal section 66, with a gradually decreasing section or opening. Said tapered section 66 ends in a circular opening 68, in correspondence with the tapered channel 52 and in proximity of the first extremity 26 of the active portion 22. The truncated conical surface of the tapered extremity 66 is the contact surface. For example, said seat 62 houses a light source or pencil light 64 or pen light, of known type, fitted with an on/off button 65 and with a light bulb 70 poking out of the circular opening 68. The pencil light source 64 is locked in the working position by hooks housed in a seat in the grip. For example the light source has an elastic extension 71 fitted with a protuberance or terminal stalk, which can be clipped into the seat 73 provided on the outside of the wall of the grip 48. Nerves 72 extend longitudinally and externally along the sides of the grip 48, uniting on the edge 58 of the section of tapered channel 52 (Figures 2 and 3).

The valve 20 is in biocompatible synthetic material and preferably in plastic for foodstuffs, for example of the type defined by the Italian Ministerial Decree dated 21.03.1973. A suitable material is for example the polystyrene ARL, preferably transparent. Said valve is constructed for example by injection moulding.

The valve 20 can be associated with a cylindrical or conical inserter 69 of known type. Said inserter 69 has a pointed or ogival extremity which in a position associated with the valve 20, extends beyond the second extremity 28 of the channel 24 for atraumatic introduction into the anal orifice.

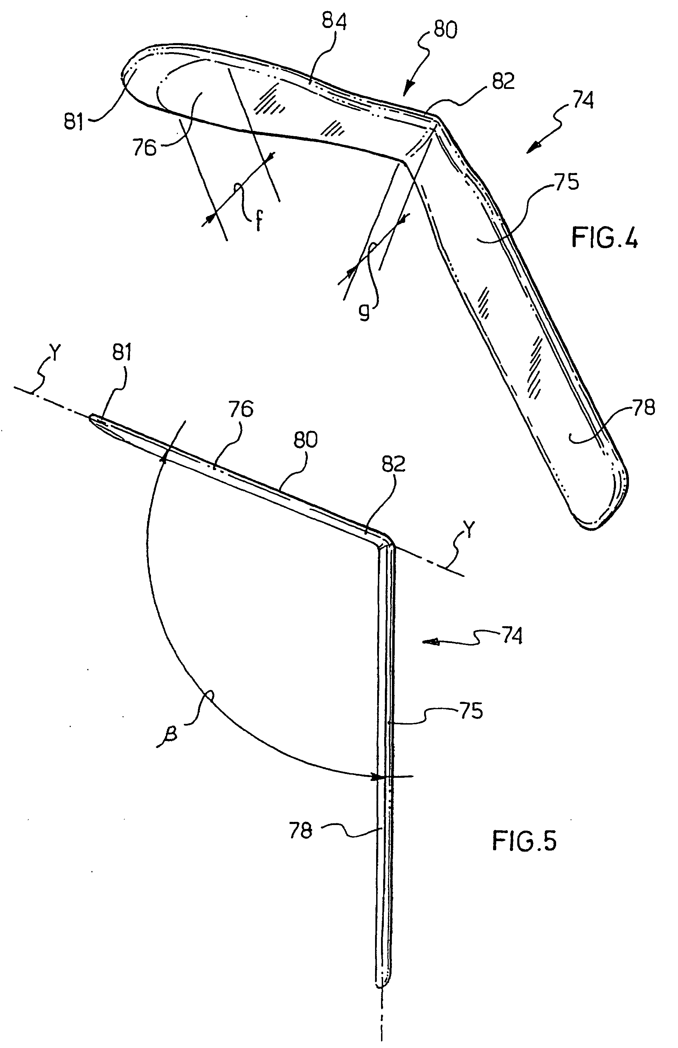

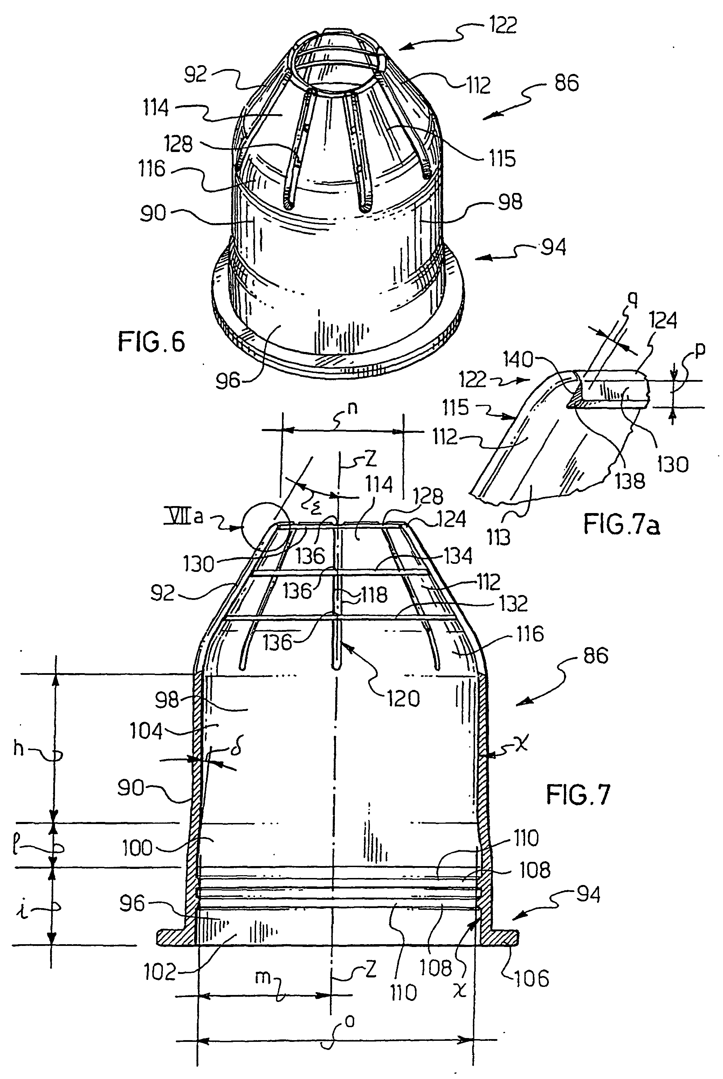

In the drawings it is also possible to note a second accessory, to be used in combination with the first accessory. Said accessory includes a depressor or spatula 74. Said spatula 74 consists of a blade 75 with an active portion 76, on axis Y - Y, joining at an angle "β" with a portion of the handle or grip 78. Said grip 78 is preferably shaped ergonomically. The angle "β" between the active portion 76 and the grip 78 distances the grip 78 from the axis Y - Y of the active part 76. For example the angle "β" varies between 100 degrees and 140 degrees and is preferably equal to 120 degrees. Said blade 75 has a slightly curved transverse section, for example, a circular arch, with concavity turned externally to the angle "β" marked by the spatula 74 or, in other words, it presents a curve on the outer edge 80 of the spatula 74 or concave side. In other words, the active portion 76 of said spatula 74 is channelled. The active portion 76 tapers towards the free extremity 81. Said active portion 76 has a width superior to the central section 82 which joins the grip 78. For example, said active portion 76 has a width "f" approximately equal to the length "d" of the active portion 22 of the valve 20 and said central section 82 has a width "g" equal to the minimum value sufficient to resist the stresses produced when the spatula 74 is used, while at the same time being of limited bulk. The spatula 74 has rounded edges 84, to avoid sharp edges. Said spatula 74 is in synthetic biocompatible material. For example, the spatula is in plastic for foodstuffs and, preferably, is in transparent ARL polystyrene (Figures 4 and 5).

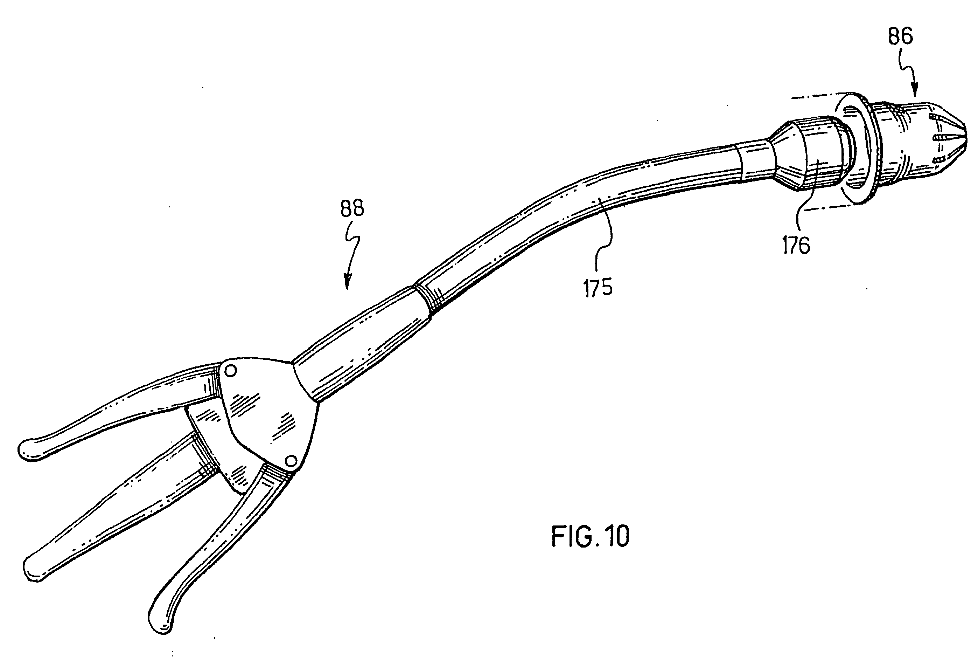

In the drawings it is also possible to note a third accessory according to the present invention which includes a mandrel or inserter 86. Said inserter 86 co-operates with a mechanical circular suturing device 88 or circular stapler, in a manner which will be described in detail below (Figure 10). Said inserter 86 is tubular sleeve shaped 90, on axis Z - Z, with a tapered section 92. The sleeve 90 has an initial section or lead-in 96 connected to a second section or annular section 98 with a smaller cross section at one free extremity 94. Said annular section 98 has an axial extension "h" variable between 10 mm and 15 mm and, preferably, equal to 13.3 mm and said lead-in 96 has an axial extension "i" variable between 8 mm and 12 mm and preferably equal to 10 mm. Said lead-in 96 and said annular section 98 are slightly conical towards the tapered section 92 and are connected by a truncated conical linking section 100. For example, the internal surface 102, 104 of the lead-in 96 and of the annular section 98 has a degree of taper "χ" variable between 0.15 degrees and 0.5 degrees and preferably equal to 0.25 degrees. The linking section 100 has a degree of taper "δ" variable between 3 degrees and 7 degrees and preferably equal to 5 degrees. Said truncated conical linking section 100 has, for example, an axial extension "1" variable between 4 mm and 8 mm and preferably equal to 6 mm. Said lead-in 96 has at the free extremity 94 a border 106 positioned transversely to the axis Z - Z of the sleeve 90. Inside the lead-in 96 there are annular ridges 108. Said ridges are elastically deformable on the axis Z - Z. Preferably, said ridges 108 have a sliding surface 110 on the extremity turned towards the axis Z - Z of the sleeve 90. Advantageously, said sliding surface 110 is positioned at a distance "m" from the axis of symmetry Z - Z of the sleeve 90 equal to the maximum distance "m" at which the internal surface 104 of the annular section 98 is positioned.

The tapered section 92 of said sleeve 90 is flower-shaped. Said flaps 112 of said flower shape have an internal surface 113 and an external surface 115 and overhang the annular section 98. The flaps 112 are elastically deformable in a direction radial to the sleeve 90 from a closed position, a bundle of converging flaps 112, to an open position a bundle of parallel flaps 112. In particular, said flaps 112 have a rectilinear terminal section 114 joined by a curved section 116, forming, with the flaps 112 in the closed position, a truncated cone mantle which reduces the lumen "n" at the extremity opposite the lead-in 96 of the sleeve 90 to a value of less than half of the lumen "o" of the lead-in 96. For example, said lead-in 96 has a maximum diameter "o" variable between 30 mm and 40 mm and preferably equal to 35.5 mm and said minimum lumen formed by the closed flaps 112 has a diameter "n" variable between 10 mm and 20 mm and preferably equal to 15 mm. Consequently the flower-shaped section has conicity "ε" preferably equal to 30 degrees. Said flaps 112, even when in a closed position, have contiguous edges 118 which do not meet, creating longitudinal divisions 120. For example, the distance between two contiguous edges 118 of the flaps 112 in the closed position varies between 0.5 mm and 1 mm and is preferably equal to 0.87 mm. The extremity 122 of said flaps 112 has a rounded edge 124, and the contiguous or lateral edges 118 of the flaps 112 are rounded in correspondence with the external surface 126 of the sleeve 90. In the closed position before use the flaps 112 are connected by bridges 128. For example, inside the flower-shaped section there is at least one ring 130, 132, and 134 which connects the flaps in the closed position. Preferably, said at least one ring is in fact three rings 130, 132, 134 positioned transversely to the axis Z - Z of the sleeve 90 at various heights of the flaps 112. A first ring 130 is positioned in proximity with the free extremity 122 of the flaps 112, a second ring 132 in proximity with the base of the flaps 112 where they are attached to the annular section 98 and a third ring 134 in correspondence with the middle of the flaps 112. Said rings 130, 132 and 134 have weak points 136 in correspondence with only one of the lateral contiguous edges 118 of the flaps 112. Said weak points 136 are constructed so that the bridges 128 will not break when the flaps 112 are strained by an action radial to the axis Z - Z of the sleeve 90. Said bridges 128 give way in correspondence with only one of the lateral contiguous edges 118 of the flaps 112, when subjected to a relatively low force radially directed towards the outside of the sleeve 90. In particular, said bridges 128 give way when they are forced by the passage of a surgical instrument, such as a mechanical circular suturing device 88, inside an inserter 86. Said rings 130, 132 and 134 have, for example, a trapezoidal transverse section 138 whose base 140 is fixed to the internal surface 113 of the flaps 112 and has an oblique side 142 positioned transversely to the axis Z - Z of the sleeve 90. Said rings 130, 132 and 134 have, for example, a section 138 at height "p" variable between 0.5 mm and 0.9 mm and preferably equal to 0.7 mm and a width "q" variable between 0.07 mm and 0.47 mm and preferably equal to 0.27 mm.

Said inserter 86 is in biocompatible synthetic material, for example, in polyethylene and, preferably, in mid-density polyethylene for foodstuffs and is constructed, for example, by injection moulding. (Figures 6, 7 and 7a).

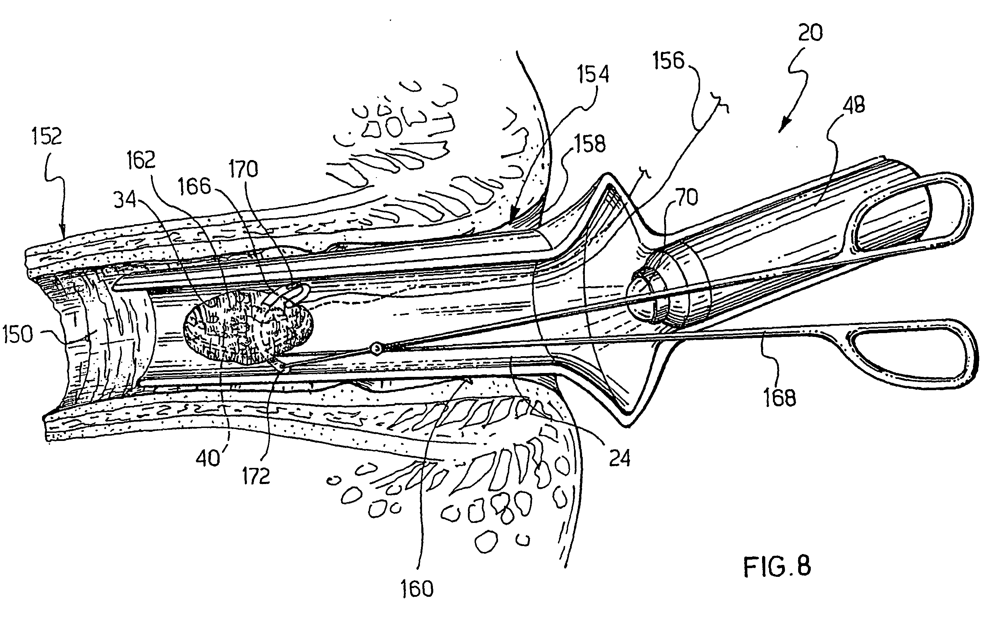

The method for using the above-described accessories 20, 74 and 90 is described below, taking as a non-limiting example an operation to remove a section of mucosa 150 of the ampulla of rectum 152 in proximity of the anal duct 154, with reference to Figures 8 to 15.

If, for example, prolapsed haemorrhoids are present, one of the operative techniques consists of removing a tubular or cylindrical section of mucosa of the ampulla of rectum. Said section must be removed at a set depth on the ampulla of rectum or, in other words, at a suitable distance from the dentate line, for example at approximately 60 mm from the anal orifice.

This operation presents the following principal stages. First of all, thanks to the fact that the mucosa can slide freely on the submucosal layer, a ring or circumferential purse-string suture is created at said depth of the mucosa. Successively the purse-string suture is closed, strangling the mucous cylinder, which positions itself transversely to the axis of the ampulla of rectum. The mucous cylinder deformed to a disc or ring shape, is then resected, simultaneously suturing the remaining overlapped rims of the mucosa.

The various surgical steps are described in more detail below.

First of all, some stages of the preparatory stages are described. First the ogival inserter 69 and the valve 20 are disconnected. Then a suture thread 156 is passed through the window 34 on the valve 20 and held across the window 34. Said suture thread 156 is passed from the convex side to the concave side of the valve 20, leaving a section of approximately 200 mm outside the valve 20. The internal portion of the suture thread 156 is positioned along the concavity of the valve 20 and then along the grip 48 of the same. The ogival inserter 69 is then repositioned in the channel 24 of the valve 20. A pencil light 64 is introduced into the seat 62 of the grip 48 until it sits against the surface of the tapered end 66, allowing the bulb 70 to poke out of the upper circular opening 68.

The stages of the operation are described below. The valve 20, fitted with the ogival inserter 69, is introduced into the anal duct 154 as far as possible, so that the valve guard 20, that is the funnel-shaped section 52, is in contact with the perineal tissue surrounding the anus 158. The ogival inserter is then removed 69. Once the desired position is reached, thanks to the positioning of the bulb 70 of the light 64 in proximity with the first extremity 26 of the channel 24, it is possible to attain perfect illumination of the operative area and at the same time free access, not obstructed by the means of illumination 64. Access to the operative area is particularly facilitated by the wings 54 and 56 of the funnel-shaped section 52, which hold the sections of anal duct 154 apart. Once the ogival inserter 69 has been removed the spatula 74 which holds the mucosa 150 opposite the valve 20 away from the operative field is introduced into the ampulla of rectum. The position of the grips 48 and 78 of the valve 20 and the spatula 74 at an angle with respect to the relative active portions 22 and 76, makes it possible to maintain the active portions 22, 76 in the correct position, manipulating the accessories far from the visual field. Once the external section of the suture thread 156 is fixed to the operative field, one proceeds with light movements of the handle 48 of the valve 20, causing an extrusion of rectal mucosa 150 in the window 34 at a suitable distance from the dentate line 160. To evaluate whether the window 34 is positioned at the correct depth, it is possible to use the graduated scale 44 on the valve 20. When the mucosa 150 is seen to be extruded or introflexed through the window 34 of the valve 20, a portion of mucosa 162 is integrally transfixed with a suture stitch 164 from right to left, using a suture thread 156 fitted with an atraumatic needle 166. This operation is carried out by manoeuvring the needle 166 with the needle holder 168, of known type, in proximity with its eye 170. The left portion of the channel 24 of which the valve 20 is constructed, and therefore the edge or border 40 marking the window 34 itself, are used as a rest for the tip 172 of the needle 166 which pokes out of the transfixed portion of mucosa 162. In this way, the point 172 of the needle 166 is easily identifiable and can be recovered using the needle holder 168 (Figure 8). The suturing procedure is then completed, with the suture thread 156 still across the window 34 of the valve 20, for the entire circumference of the ampulla of rectum 152, moving the valve 20 at an angle and the counterpoised spatula 74 at an angle sufficient to cause a new portion of mucosa of the circumferential section of the ampulla of rectum 152 to extrude into the window 34 at the same depth. Once the suturing is completed, the valve 20 and the spatula 74 are removed and a digital check is made of the circumferential completeness of the purse-string suture 174 (Figure 9). This stage of the operation is completed by cutting the needle 166 and blocking the two ends of the suture thread 156 with a clip.

The stages relative to the preparation and use of the mechanical circular suturing device 88 are described below. Once the purse-string suture 174 is completed it is necessary to introduce the surgical instruments necessary for the removal of the section of mucosa adjacent to the purse-string suture 174 and for the suturing of the remaining circumferential flaps. Said operation is carried out using a mechanical circular suturing device 88, of known type, which has at the operative extremity of a stalk 175, an anvil 176 housing a cylindrical blade 178 and a device for shooting in the clips 180. A disc or head 182 co-operates with said anvil 176 shaped ogivally at the top and controlled by means of a jointed rod 184, or control rod, distancing (open position) and approaching (closed position) the anvil 176, in order to close the clips 180 during shooting or introduction of the clips in the tissues. Said anvil 176 and said head 182 form the cutter of the suturing device 88.

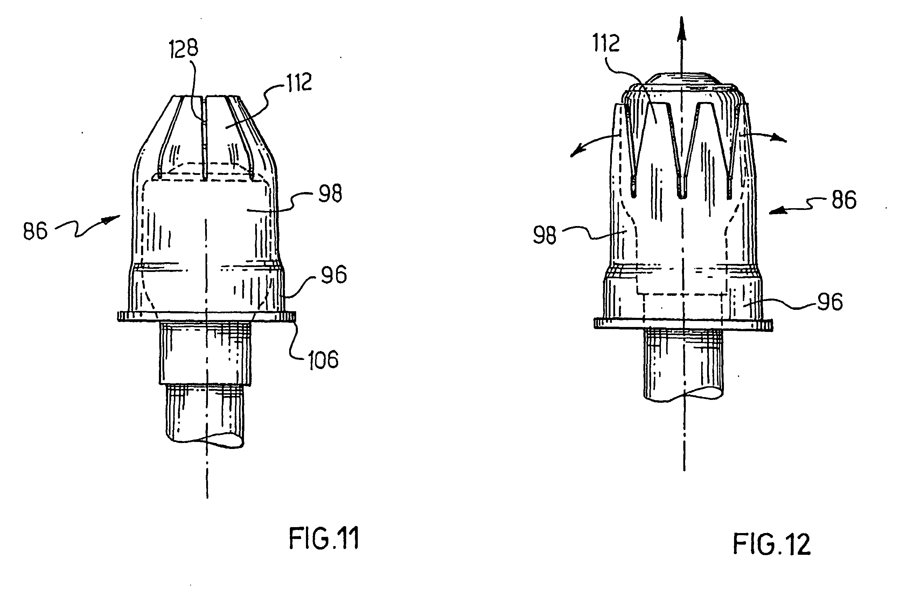

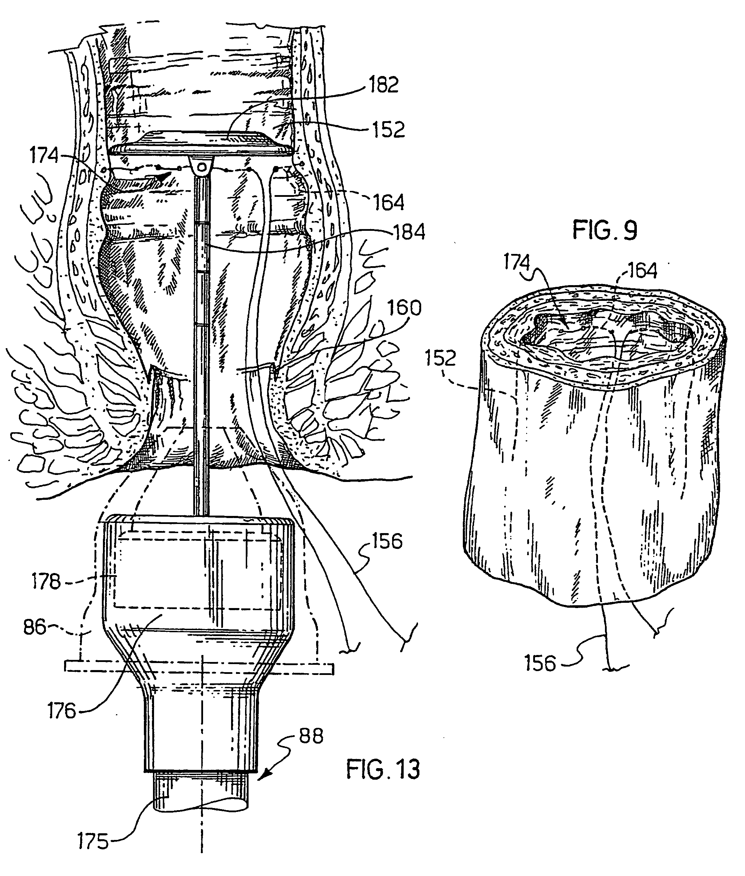

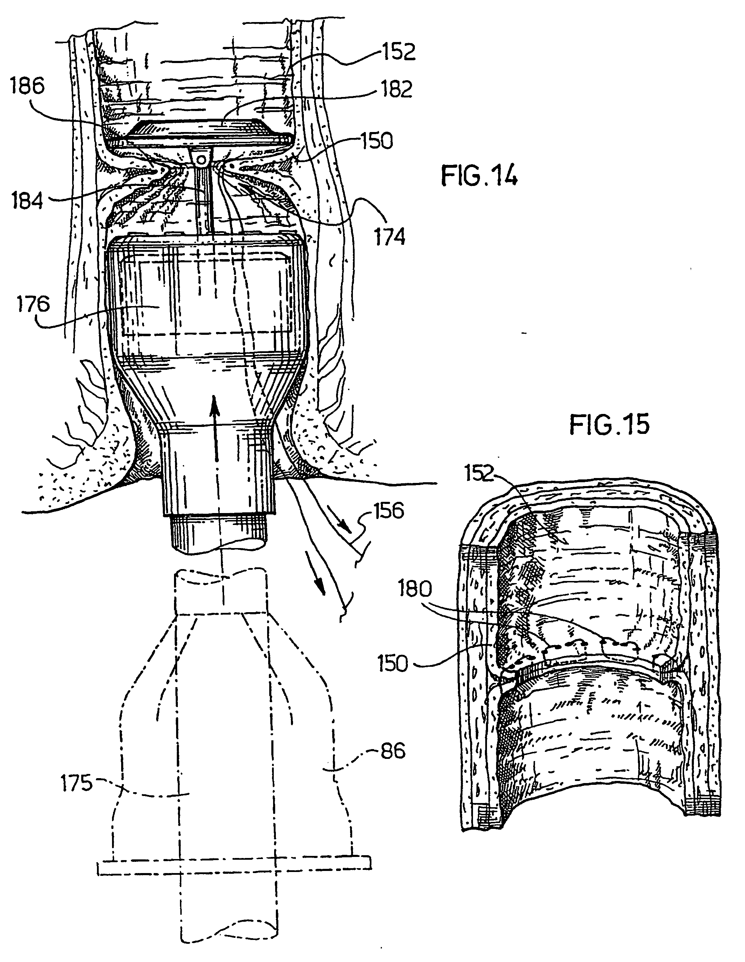

First of all, the suturing device 88 is opened completely and the head 182 is removed. Then the flower-shaped inserter 86 is fitted on the anvil 176 of the suturing device 88. The head 182 is then replaced, the suturing device 88 being opened as far as possible. The head 182 is introduced into the anal duct 154, ensuring that the head 182 overcomes the obstacle of the purse-string suture 174 previously stitched (Figure 13). The two ends of the suture thread 156 are knotted and a check is made to ensure that the mucosa 150 is positioned around the control rod 184 of the suturing device 88. In particular, on tightening the purse-string 174 around the stalk of the control rod 184, the mucosa 150 will stick around the stalk 184. In other words, the mucosa 150 will be introflexed positioning itself in such a way as to form an annular disc 186. To do this the ends of the suture thread 156 are pulled and knotted until they are in a strangulated position around the control rod 184 (Figure 14). Later it is necessary to bring the anvil 176 in proximity with the head 182. To do this it is necessary to introduce the anvil into the anal duct 154. Since the anvil 176 does not have a rounded ogival form it is possible that during this operation the very delicate epithelium of the anus or the perineal tissue surrounding the anus may be damaged. Therefore the tapered section 92 and the annular section 98 of the inserter 86 are inserted into the anal duct. Thanks to the bridges 128 this operation is carried out without deforming the tapered section 92, avoiding disordering of the gathered position of the perfectly truncated conical flaps 112. It is then possible to tighten the cutting of the suturing device 88. The anvil 176 breaks the bridges 128 and passes through the flaps 112 moving into the ampulla of rectum 152. The anvil 176 is placed beside the head 182 tightening the annular disc 186 of mucosa 150 (Figures 12 and 14).

Once the cutting of the suturing device 88 has been tightened, the flower-shaped inserter 86 is withdrawn from the anal duct 154 and positioned on the stalk 175 of the suturing device 88.

Successively the annular disc 186 is cut withdrawing the cylindrical blade 178 and simultaneously shooting metal clips 180 into the rims or borders folded and laid against the remaining mucosa 150 (Figure 15).

Once the suturing has been carried out the suturing device 88 is removed and the rim of the suturing is checked, positioning, by means of the ogival inserter 69, the valve 20 with the window 34 in correspondence with the suture itself, rotating it as necessary. Finally the valve 20 is removed.

It is clear that variations and/or additions may be made to the procedures described and illustrated above. The grip 48 of the valve 20 may be constructed as a channel, equipped with ridges for a notch coupling with the means of illumination 64.

At the extremity of the grip 48, facing the circular opening 68 housing the bulb 70 of the light, it is possible to foresee guides for the beam of light, capable of orienting said beam according to the axis X - X of the channel 24. Said means of illumination are for example formed of a bar to guide the light 188 (Figure 2). Said bar 188 is curved and conveys the light from the bulb 70 of the light on the first extremity 26 of the channel 24. Said bar 188 is part of the body of the valve 20. In this case the ogival inserter 69 which can be associated is fitted with a channel that, in the working position, holds the bar 188.

With reference to the inserter, the length of the annular section can vary according to the depth at which the operative extremity of the surgical instrument is positioned. In this way the non-rounded extremity of the instrument does not damage the section of intestinal tract to be passed through. During introduction of the inserter the truncated cone tapered section gradually and atraumatically widens the wall of the intestinal tract to be passed through.

All the accessories described are disposable and are therefore used for only one operation.

As will be appreciated from the above description an inserter with an annular section and a tapered section makes it possible to insert a surgical instrument into the intestinal tract atraumatically. In particular, the fact that the rounded or ogival extremity of the surgical instrument does not damage the perineal tissue surrounding the anus and the wall of the intestinal tract to be passed through in order to reach the depth for the operation, is an advantage.

The fact that the tapered section is deformable for the passage of the operative extremity of the surgical instrument is particularly advantageous.

It is also advantageous that the tapered section has a number of flaps and in particular that said flaps form a truncated conical and not an ogival element, since this shape makes it possible to widen the anal duct gradually and in a safely controlled manner, avoiding traumatic introduction of the instrument. The numerous flaps advantageously maintain their truncated conical shape during insertion into the anus thanks to the bridges. In particular, the at least one ring connected to the inside of the flaps makes the tapered section resistant to the external stresses to the inside of the flaps, avoiding the disordering of the bundle of flaps during insertion and, therefore, traumatic deformation of the anal duct.

A further advantage comes from the rim of the inserter which is both a guard which prevents the inserter being pushed too far into the anal duct, and an element which makes it possible to grasp the inserter for easy and rapid withdrawal from the anal duct.

Another advantage is the inclusion of ridges in the lead-in of the inserter. The ridges serve the purpose of guiding the surgical instrument to be inserted into the anal duct. During this phase said instrument runs on the sliding surface of the ridges of limited extension and therefore capable of resisting some sliding, allowing perfect control of the introduction of the instrument and atraumatic action.

Another advantage of the invention is that the inclusion of an inserter in synthetic material makes it possible to simplify the construction, and thanks to the reduced weight of the instrument storage and transportation are simplified.

Advantageously the inserter is a disposable or single-use device.

The inserter proposed also allows the painless insertion into the intestine of a transanal access surgical instrument other than the mechanical circular suturing device previously described and may be used in operations other than that described above.

## Claims

1. Accessory for a transanal access surgical instrument and, in particular, for a mechanical circular suturing device (88), including a sleeve-shaped (90) inserter (86), with an annular section (98), into which the operative extremity of said surgical instrument slides, and a tapered section (92), for the painless introduction of said operative extremity in an intestinal tract;
said tapered section (92) being flower-shaped with a group of flaps (112) which open like a flower when said operative extremity passes through;
said group of flaps (112) being fixed overhanging the annular section (98) of the sleeve (90) and are elastically deformable in the direction radial to the sleeve (90) between a closed position, with the flaps (112) gathered in a converging bundle, and an open position to create an opening through which the operative extremity can pass;
**characterized in that**
in the closed position the flaps (112) are linked together by means of bridges (128).

2. Accessory, according to Claim 1, **characterized in that** said bridges (128) have a weak point (136) in correspondence with only one of the contiguous edges (118) of the flaps (112).

3. Accessory, according to Claim 1, **characterized in that** at least one ring (130, 132, 134) is foreseen inside the tapered section (92) for connecting the flaps (112) in the closed position.

4. Accessory, according to Claims 1, **characterized in that** the tapered section (92) is a truncated cone.

5. Accessory, according to Claim 4, **characterized in that** said truncated conical tapered section (92) has conicity (ε) equal to 30 degrees.

6. Accessory, according to Claim 4, **characterized in that** every flap (112) has a rectilinear section (114) which extends from a curved section (116) in turn connected to the annular section (98).

7. Accessory, according to Claim 1, **characterized in that** a longitudinal slit (120) is foreseen between contiguous flaps (112).

8. Accessory, according to Claim 1, **characterized in that** the edges (118, 124) of said flaps (112) are rounded.

9. Accessory, according to Claim 1, **characterized in that** at one extremity (94) opposite the tapered section (92) there is a lead-in (96) connected to the annular section (98) by means of a truncated conical connecting section (100).

10. Accessory, according to Claim 9, **characterized in that** said lead-in (96) and said annular section (98) have conicity (χ) towards the tapered section.

11. Accessory, according to Claim 9, **characterized in that** said lead-in (96) internally has annular ridges (108).

12. Accessory, according to Claim 11, **characterized in that** said ridges (108) have a sliding surface (110) at the free extremity.

13. Accessory, according to Claim 1, **characterized in that** said sleeve (90) has at the extremity (94) opposite the tapered section (92) a radial rim (106).

14. Accessory, according to Claim 1, **characterized in that** said inserter (86) is in synthetic biocompatible material.

15. Accessory, according to Claim 14, **characterized in that** said inserter (86) is in polyethylene.

## Patentansprüche

1. Zubehör für ein transanal-invasiv zu verwendendes chirurgisches Instrument und insbesondere für ein mechanisches Rundnahtgerät (88), umfassend einen hülsenförmige (90) Inserter (86) mit einem ringförmigen Querschnitt (98), in welchen das äußerste Ende des chirurgischen Instruments gleitet, und einen verjüngten Abschnitt (92) für die schmerzlose Einführung des äußersten Endes in einen Darmtrakt;
wobei der verjüngte Abschnitt (92) blumenförmig ist mit einer Gruppe von Klappen (112), die sich wie eine Blume öffnen, wenn das äußerste Ende hindurch läuft;
wobei die Gruppe von Klappen (112) so befestigt ist, dass diese über den ringförmigen Abschnitt (98) der Hülse (90) hinausragen und in der Richtung radial zur Hülse (90) zwischen einer geschlossenen Position, bei der die Klappen (112) zu einem konvergierenden Bündel zusammenlaufen, und einer offenen Position elastisch verformbar sind, um eine Öffnung zu erzeugen, durch welche das äußerste Ende verlaufen kann;
**dadurch gekennzeichnet, dass** in der geschlossenen Position die Klappen (112) mittels Verbindungsstegen (128) miteinander verbunden sind.

2. Zubehör nach Anspruch 1, **dadurch gekennzeichnet, dass** die Verbindungsstege (128) einen Schwachpunkt (136) aufweisen, der mit nur einer der angrenzenden Kanten (118) der Klappen (112) übereinstimmt.

3. Zubehör nach Anspruch 1, **dadurch gekennzeichnet, dass** zumindest ein Ring (130, 132, 134) innerhalb des verjüngten Abschnitts (92) vorgesehen ist, um die Klappen (112) in der geschlossenen Position zu verbinden.

4. Zubehör nach Anspruch 1, **dadurch gekennzeichnet, dass** der verjüngte Abschnitt (92) ein Kegelstumpf ist.

5. Zubehör nach Anspruch 4, **dadurch gekennzeichnet, dass** der kegelstumpfförmig verjüngte Abschnitt (92) eine Konizität (ε) gleich 30 Grad aufweist.

6. Zubehör nach Anspruch 4, **dadurch gekennzeichnet, dass** jede Klappe (112) einen geradlinigen Abschnitt (114) aufweist, der sich von einem gekrümmten Abschnitt (116), der wiederum mit dem ringförmigen Abschnitt (98) verbunden ist, erstreckt.

7. Zubehör nach Anspruch 1, **dadurch gekennzeichnet, dass** ein länglicher Schlitz (120) zwischen benachbarten Klappen (112) vorgesehen ist.

8. Zubehör nach Anspruch 1, **dadurch gekennzeichnet, dass** die Kanten (118, 124) der Klappen (112) gerundet sind.

9. Zubehör nach Anspruch 1, **dadurch gekennzeichnet, dass** an einem äußersten Ende (94) gegenüber dem verjüngten Abschnitt (92) eine Einführvorrichtung (96) vorhanden ist, die mit dem ringförmigen Abschnitt (98) mittels eines kegelstumpfförmigen Verbindungsabschnitt (100) verbunden ist.

10. Zubehör nach Anspruch 9, **dadurch gekennzeichnet, dass** die Einführvorrichtung (96) und der ringförmige Abschnitt (98) eine Konizität (χ) in Richtung zum verjüngten Abschnitt aufweisen.

11. Zubehör nach Anspruch 9, **dadurch gekennzeichnet, dass** die Einführvorrichtung (96) innen ringförmige Rippen (108) aufweist.

12. Zubehör nach Anspruch 11, **dadurch gekennzeichnet, dass** die Rippen (108) eine gleitende Oberfläche (110) an dem freien äußersten Ende aufweisen.

13. Zubehör nach Anspruch 1, **dadurch gekennzeichnet, dass** die Hülse (90) am äußersten Ende (94) gegenüber dem verjüngten Abschnitt (92) einen radialen Rand (106) aufweist.

14. Zubehör nach Anspruch 1, **dadurch gekennzeichnet, dass** der Inserter (86) aus einem synthetischen biokompatiblen Material ist.

15. Zubehör nach Anspruch 14, **dadurch gekennzeichnet, dass** der Inserter (86) aus Polyethylen ist.

## Revendications

1. Accessoire pour instrument chirurgical d'accès transanal et, particulièrement, pour un dispositif (88) de suturage circulaire mécanique, comprenant un élément d'insertion (86) en forme de manchon (90), une section annulaire (98) dans laquelle l'extrémité fonctionnelle dudit instrument chirurgical coulisse, et une section effilée (92) destinée à l'insertion indolore de ladite extrémité fonctionnelle dans une voie intestinale ;
ladite section effilée (92) ayant une forme de fleur pourvue d'un groupe de volets (112) qui s'ouvrent à la manière d'une fleur lorsqu'elle est traversée par ladite extrémité fonctionnelle ;
ledit groupe de volets (112) étant fixé en porte-à-faux de la section annulaire (98) du manchon (90) et pouvant se déformer élastiquement dans la direction radiale au manchon (90) entre une position fermée, les volets (112) étant groupés en un faisceau convergeant, et une position ouverte servant à créer une ouverture que peut traverser l'extrémité fonctionnelle ;
**caractérisé en ce que**
dans la position fermée, les volets (112) sont liés les uns aux autres au moyen de ponts (128).

2. Accessoire selon la revendication 1, **caractérisé en ce que** lesdits ponts (128) comportent un point faible (136) en correspondance avec seulement l'un des bords contigus (118) des volets (112).

3. Accessoire selon la revendication 1, **caractérisé en ce qu'**au moins un anneau (130, 132, 134) est prévu à l'intérieur de la section effilée (92) à des fins de liaison des volets (112) dans la position fermée.

4. Accessoire selon la revendication 1, **caractérisé en ce que** la section effilée (92) est un cône tronqué.

5. Accessoire selon la revendication 4, **caractérisé en ce que** ladite section effilée à cône tronqué (92) a une conicité (ε) égale à 30 degrés.

6. Accessoire selon la revendication 4, **caractérisé en ce que** chaque volet (112) comporte une section rectiligne (114) qui s'étend à partir d'une section courbée (116) reliée, à son tour, à la section annulaire (98).

7. Accessoire selon la revendication 1, **caractérisé en ce qu'**une fente longitudinale (120) est prévue entre des volets contigus (112).

8. Accessoire selon la revendication 1, **caractérisé en ce que** les bords (118, 124) desdits volets (112) sont arrondis.

9. Accessoire selon la revendication 1, **caractérisé en ce qu'**un orifice d'entrée (96) lié à la section annulaire (98) au moyen d'une section à cône tronqué (100) de liaison est réalisé au niveau d'une extrémité (94) opposée à la section effilée (92).

10. Accessoire selon la revendication 9, **caractérisé en ce que** ledit orifice d'entrée (96) et ladite section annulaire (98) ont une conicité (χ) orientée vers la section effilée.

11. Accessoire selon la revendication 9, **caractérisé en ce que** ledit orifice d'entrée (96) comporte des nervures annulaires intérieures (108).

12. Accessoire selon la revendication 11, **caractérisé en ce que** lesdites nervures (108) comportent une surface (110) de glissement au niveau de l'extrémité libre.

13. Accessoire selon la revendication 1, **caractérisé en ce que** ledit manchon (90) comporte un rebord radial (106) au niveau de l'extrémité (94) opposée à la section effilée (92).

14. Accessoire selon la revendication 1, **caractérisé en ce que** ledit élément d'insertion (86) est en matériau synthétique biocompatible.

15. Accessoire selon la revendication 14, **caractérisé en ce que** ledit élément d'insertion (86) est en polyéthylène.
